# EUROPÄISCHE PATENTSCHRIFT

(11) **EP 1 340 072 B1**
(45) Veröffentlichungstag und Bekanntmachung des Hinweises auf die Patenterteilung: **02.06.2010**
(21) Anmeldenummer: 01967301.1
(22) Anmeldetag: 30.08.2001
(51) Int. Cl.: G01N 31/22, G01N 33/18

(54) **VERFAHREN UND MITTEL ZUR BESTIMMUNG VON GESAMTHÄRTE**
METHOD AND AGENT FOR DETERMINING TOTAL WATER HARDNESS
PROCEDE ET AGENT PERMETTANT DE DETERMINER UN TITRE HYDROTIMETRIQUE

(30) Priorität: 08.12.2000 DE 10061140
(43) Veröffentlichungstag der Anmeldung: 03.09.2003
(73) Patentinhaber: Merck Patent GmbH, 64293 Darmstadt (DE)
(72) Erfinder: KRENN, Karl-Dieter, 64319 Pfungstadt (DE); TANZER, Dieter, 64372 Ober-Ramstadt (DE)
(86) Internationale Anmeldenummer: PCT/EP2001/009967
(87) Internationale Veröffentlichungsnummer: WO 2002/046742

(56) Entgegenhaltungen:
- EP-A- 0 254 202
- EP-A- 0 798 563
- DE-A- 19 623 747
- JP-A- 01 025 061
- JP-A- 55 000 426
- JP-A- 62 064 951
- US-A- 3 822 116
- DATABASE WPI Section Ch, Week 8703 Derwent Publications Ltd., London, GB; Class B04, AN 1987-018625 XP002185556 -& JP 61 277057 A (SHIMADZU CORP), 8. Dezember 1986 (1986-12-08)

## Beschreibung

Die Erfindung betrifft ein trockenchemisches Verfahren und einen Teststreifen zum schnellen, quantitativen Nachweis von Calcium und Magnesium in wässrigen Proben.

Das in der Natur vorkommende Wasser einschließlich unseres Trinkwassers enthält neben verschiedenen Gasen eine Reihe von Salzen und anderen Verbindungen, die aus den Böden und Gesteinen herausgelöst werden. Von besonderer Bedeutung sind dabei die Härtebildner des Wassers. Unter der Gesamthärte versteht man die im Wasser gelösten Erdalkalimetallionen, im wesentlichen handelt es sich dabei um Calcium (Ca)- und Magnesium (Mg)-Salze. Strontium- und Barium kommen nur in vernachlässigbaren Spuren vor. Etwa 70 - 85 % der Gesamthärte sind auf den Ca-Gehalt und etwa 15 - 30 % auf den Mg-Gehalt der Wässer zurückzuführen.

Die Härte des Wassers wird in Deutschland in deutschen Härtegraden (°dH) angegeben, wobei die Härtegrade als CaO berechnet werden. Dabei entspricht definitionsgemäß 1°dH einem Gehalt von 10 mg CaO/l oder 0,178 mmol CaO/l oder 7,14 mg MgO/l Wasser (z.B. K. Höll; Wasser, 7. Auflage (1986), Walter de Gruyter, Berlin)).
Wässer mit Härtegraden von 0 - 7 °dH werden als weich (Härtebereich 1), mit Härtegraden von 8 -14 °dH als mittelhart (Härtebereich 2), mit Härtegraden von 15 - 20°dH als hart (Härtebereich 3) und mit Härtegraden über 21°dH als sehr hartes Wasser (Härtebereich 4) bezeichnet.

Weiche Wässer finden sich in niederschlagsreichen Gegenden mit Gesteinen geringer Löslichkeit, harte Wässer finden sich vor allem in niederschlagsarmen Gebieten mit Gips- und Kalk-Gesteinen. Auch die jahreszeitlich bedingten Schwankungen sind dabei nicht unerheblich.

Bei vielen industriellen und gewerblichen Prozessen darf das Wasser keine oder nur geringe Härte aufweisen, weshalb der Gesamthärte-Gehalt regelmäßig überprüft werden muß.

Als wesentliches Qualitätskriterium des Wassers beeinflußt die Wasserhärte den Geschmack von Speisen und Getränken (z.B. Kaffee, Wein, Bier, Mineralwässer). Darüber hinaus wird auch ein Einfluss der Wasserhärte auf die Gesundheit des Menschen diskutiert. Im allgemeinen gilt eher zu weiches als zu hartes Wasser als gesundheitsgefährdend, da es Schwermetallspuren z.B. aus Rohrleitungen leichter zu mobilisieren vermag und einen nachteiligen Einfluß auf die Knochen- und Zahnbildung hat.

Der Bestimmung der Gesamthärte von Wasser kommt daher in der Lebensmittelanalytik und Umweltanalytik ein wichtige Bedeutung zu.

Die Bestimmung der Gesamthärte erfolgt vorwiegend durch massanalytische Verfahren (Titrationen). Gemäß DIN 38409 Teil 6 wird die Gesamthärte komplexometrisch mit Titriplex^{®} III-Lösung (Ethylen-dinitrilotetraessigsäure Dinatriumsalz) gegen eine Indikatorpuffertablette bestimmt.

Häufig wird auch der Gehalt an Calcium und Magnesium mittels spektroskopischer Verfahren (z.B. Atomabsorption, Flammenphotometrie) bestimmt und daraus der Gehalt an Gesamthärte berechnet (z.B. L.A. Hütter, Wasser und Wasseruntersuchung, 4. Auflage (1990) Verlag Sauerländer, Aarau).

Verfügbare Verfahren zur Schnellbestimmung von Gesamthärte beruhen auf dem titrimetrischen Bestimmungsverfahren. Aufgrund der Bestimmungsgenauigkeit werden dazu meist sogenannte naßchemische Testsätze herangezogen. Eine abgemessene Menge an Probe wird dabei mit einem Indikator versetzt und das Titrationsmittel mittels einer geeigneten Dosiereinrichtung (z.B. Pipette) zugegeben bzw. zugetropft. Die Menge an verbrauchtem Titrationsmittel ist ein Maß für die Gesamthärte der Probe und kann z.B. über eine Skalierung auf der Pipette bzw. über die Anzahl an Tropfen zugeordnet werden.

Weiterhin sind naßchemische Verfahren zur Bestimmung von Calcium und Magnesium bekannt, die nicht auf einem titrimetrischen Verfahren beruhen, sondern als farbgebendes Reagenz (Indikator) den Säure-Base-Indikator Orthokresolphtalein-Komplexon (o-CPC: 3',3"-Bis[(bis-(carboxymethyl)-amino)-methyl]-5',5'-dimethylphenolphtalein - auch als Phtaleinpurpur bezeichnet) verwenden. Insbesondere wird dieser Farbstoff zur Bestimmung von Ca in Körperflüssigkeiten herangezogen.

Beispiele finden sich in DE 2335350, EP 0 203 334 sowie W.R. Moorehead und H.G. Biggs; Clin. Chem. 20 (1974), 1458 - 1460.

Nachteil dieser bekannten Verfahren ist, dass sie nicht für eine quantitative parallele Bestimmung von Ca und Mg herangezogen werden können.

Zudem sind die Methoden umständlich und erfüllen nicht die Ansprüche, die der Kunde an eine einfache Bestimmungsmethode für Gesamthärte stellt, da Gesamthärtebestimmungen häufig von ungeschulten, fachfremden Personen durchgeführt werden. Es müssen beispielsweise Reagenzienlösungen verwendet werden, die nach der Bestimmung geeignet entsorgt werden müssen.

In jüngerer Zeit hat die Analyse mit festen, saugfähigen Trägern, den sogenannten Teststäbchen, zunehmend an Bedeutung gewonnen. Zu den wesentlichen Vorteilen dieser trockenchemischen Verfahren gehören insbesondere die einfache Handhabung sowie die aufgrund der geringen Reagenzmengen unproblematische Entsorgung. Alle oder der größte Teil der für die Nachweisreaktion notwendigen Reagenzien sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge des zu bestimmenden Analyten und kann visuell, d.h. halbquantitativ, bzw. quantitativ mit einfachen Reflektometern ausgewertet werden.

Teststäbchen zur Bestimmung von Gesamthärte sind im Handel erhältlich. Der Nachweis basiert ebenfalls auf den oben bereits erwähnten titrimetrischen Verfahren. Die Teststäbchen müssen für die Bestimmung nur in die Probe eingetaucht werden. Auf derartige Teststäbchen sind mehrere Nachweiszonen aufgebracht, die neben anderen für die Nachweisreaktion notwendigen Reagenzien unterschiedliche Mengen an Titrationsmittel (Titriplex^{®} III) enthalten. In Abhängigkeit von der Härte des zu untersuchenden Wassers und der Menge an Titrationsmittel kommt es zu einem definierten Farbumschlag auf den Testzonen.

Wesentlicher Nachteil dieser Teststäbchen ist, daß, bedingt durch das Nachweisprinzip, nur die Größenordnung der Gesamthärte bestimmt werden kann. Für eine quantitative Analyse mit Hilfe eines Reflektometers kann diese Art des Nachweises nicht herangezogen werden. Dazu bedarf es einer kontinuierlichen Veränderung der Farbtiefe auf dem Teststäbchen, d.h. der Abnahme bzw. Zunahme der Remission bei einer bestimmten Wellenlänge.

Die Herstellung dieser Teststäbchen ist zudem sehr umständlich, da mehrere mit unterschiedlichen Mengen an Reagenzien imprägnierte Zonen auf einen Träger aufgesiegelt werden müssen.

Nachteilig an dem bekannten Teststäbchennachweis für Gesamthärte ist insbesondere auch die Schwierigkeit der Zuordnung der Reaktionszonen zur Farbskala, da die entstehende Färbung auf den Testzonen nicht stabil ist und sich kontinuierlich verstärkt.

Ungeeignet ist das existierende Nachweisprinzip ebenfalls für die Herstellung von Mehrfachteststreifen, bei denen mehrere Nachweiszonen für verschiedene Parameter auf einem Teststreifen aufgebracht werden müssen. Diese Form der Teststreifen finden besonders in der Diagnostik und im Aquaristik-Bereich Anwendung.

Der vorliegenden Erfindung liegt daher die Aufgabe zu Grunde, ein Verfahren zur Bestimmung von Gesamthärte in wäßrigen Proben zur Verfügung zu stellen, das obige Nachteile nicht aufweist, eine einfache und schnelle Ausführung ermöglicht und kostengünstig ist. Das Verfahren sollte auch eine Integration von Gesamthärte in Mehrfachteststreifen ermöglichen. Insbesondere sollte das Verfahren nicht nur einer halbquantitativen, visuellen, sondern auch einer quantitativen Auswertung mit einem Reflektometer zugänglich sein.

Es wurde gefunden, daß ein Verfahren zur Bestimmung von Gesamthärte mittels eines Teststreifens, der Orthokresolphtalein-Komplexon als farbgebendes Reagenz in einem neutralen bis sauren Puffer aufweist, alle genannten Anforderungen erfüllt. Teststreifen zum Nachweis von Calcium unter Verwendung von Orthokresolphtalein-Komplexon als farbgebendes Reagenz sind aus JP 55 000 426 A und JP 1 025 061 A bekannt. Weiterhin offenbart JP 62 064 951 A eine parallele Bestimmung von Calcium und Magnesium durch Einstellung der Empfindlichkeit von Orthokresolphtalein-Komplexon.

Gegenstand der vorliegenden Erfindung ist daher ein Verfahren zur Bestimmung der Summe aus Calcium und Magnesium in wässrigen Proben, gemäß Anspruch 1.

In einer bevorzugten Ausführungsform liegt der pH-Wert des Puffersystems bei kleiner oder gleich pH 7.

Gegenstand der vorliegenden ist auch ein Teststreifen zum Nachweis von Ca und Mg gemäß Anspruch 3.

In einer bevorzugten Ausführungsform besteht das Puffersystem aus einem Imidazolpuffer, pH 6,75.

In einer bevorzugten Ausführungsform enthält zumindest eine Zone des Teststreifens zusätzlich ein Bariumsalz.

In einer anderen bevorzugten Ausführungsform weist zumindest eine Zone des Teststreifens einen Ca- oder Mg-spezifischen oder einen unspezifischen Komplexbildner auf.

In einer bevorzugten Ausführungsform weist der Teststreifen zusätzlich zu mindestens einer Zone zum erfindungsgemäßen Nachweis von Ca und Mg weitere Zonen zum Nachweis anderer Analyten, wie z.B. Nitrit, Nitrat, pH-Wert oder Carbonathärte, auf.

Gegenstand der vorliegenden Erfindung ist auch ein Analysekit zum Nachweis von Ca und Mg in wässrigen Proben, der zumindest einen erfindungsgemäßen Teststreifen enthält.

Das erfindungsgemäße Verfahrens bzw. der erfindungsgemäße Teststreifen sind insbesondere geeignet zum Nachweis der Gesamtmenge an Ca und Mg in wässrigen Proben, wie Wasserproben, Lebensmitteln oder Körperflüssigkeiten. Besonders bevorzugt dient das erfindungsgemäße Verfahren zur Analyse von Wasser- und Lebensmittelproben. Bei Lebensmittelsproben ist zu beachten, daß oftmals ein gewisser Anteil des nachzuweisenden Ca und/oder Mg in gebundener Form vorliegt. In diesem Fall muß die Probe zunächst geeignet aufgeschlossen werden.

Basis des erfindungsgemäßen Verfahrens ist die Verwendung des farbgebenden Reagenzes Orthokresolphtalein-Komplexon (o-CPC: 3',3"-Bis[(bis-(carboxymethyl)-amino)-methyl]-5',5'-dimethylphenolphtalein) in Kombination mit einem Puffer mit pH < 8,5.

Das in dem erfindungsgemäßen Verfahren verwendete Nachweissystem liegt in Form eines Teststreifens d.h. einer imprägnierten Matrix vor, wobei alle für den Nachweis notwendigen Reagenzien (farbgebendes Reagenz, Puffersystem, ggf. auch Stabilisatoren und Lösungsvermittler) in einen auf dem Teststreifen befindlichen saugfähigen Träger eingebettet sind. Zumeist bedeckt der saugfähige Träger, auf den die Nachweisreagenzien aufgebracht sind nicht den gesamten Teststreifen, sondern lediglich eine Zone des Teststreifens. Auf diese Weise ist es möglich, nicht nur eine Zone mit einer Zusammensetzung von Nachweisreagenzien, sondern mehrere Zonen mit unterschiedlichen Zusammensetzungen z.B. zum Nachweis verschiedener Konzentrationsbereiche eines Analyten oder zum Nachweis unterschiedlicher Analyten auf einem Teststreifen zu kombinieren. Erfindungsgemäß wird daher der Bereich des Teststreifens, auf den die für den Nachweis eines Analyten notwendigen Reagenzien auf einen saugfähigen Träger aufgebracht sind, als Zone bezeichnet.

Alle bekannten Verfahren, bei denen Orthokresolphtalein-Komplexon als Indikator eingesetzt wird, verwenden Puffersysteme, die pH-Werte über pH 9 erzeugen. Es wurde gefunden, dass diese basischen Puffersysteme für den erfindungsgemäßen Einsatz auf einem Teststreifen nicht geeignet sind, da die alkalischen Bedingungen in Abhängigkeit des pH-Wertes zu einer mehr oder weniger tiefen Eigenfärbung ohne Anwesenheit von Ca und Mg (Blindwert) führen. Es wurde festgestellt, daß sich im Falle der Verwendung von Teststreifen unter alkalischen Reaktionsbedingungen (pH > 8,5) die Färbung des Blindwertes nur unwesentlich von der Färbung einer Probe mit hohen Gehalten an Calcium bzw. Magnesium unterscheidet.

Es wurde nun gefunden, daß eine wesentliche Voraussetzung für das Erzielen eines geringen Blindwertes unter Verwendung von o-CPC die Einstellung eines mindestens schwach alkalischen (pH <8,5), besser annähernd neutralen bzw. sogar schwach sauren pH-Wertes unter gleichzeitiger Einhaltung der notwendigen Sensitivität gegenüber Ca und Mg ist.

Für die Bestimmung der Summe aus Ca und Mg ist eine weitere wesentliche Voraussetzung die Einstellung von Bedingungen, die zu einer exakt identischen molaren Empfindlichkeit für Mg und Ca führen.

Das Puffersystem muß daher so aufeinander abgestimmt sein, daß einerseits kein bzw. nur ein sehr geringer Blindwert auftritt, andererseits eine ausreichend hohe, identische molare Empfindlichkeit bezogen auf Ca und Mg resultiert.

Geeignete Puffersysteme zur Einstellung des pH-Wertes sind solche, die auf einen saugfähigen Träger aufgebracht werden können, mit den anderen Bestandteilen des Testes verträglich sind und die Nachweisreaktion nicht stören.

Besonders bevorzugt sind Puffersysteme auf Basis von Aminen, besonders bevorzugt heterozyklischen Aminen. Beispiele für besonders geeignete Puffersysteme sind Imidazol-, 1-Methylimidazol-, 2-Methylimidazol-, Pyrazol-, Pyrimidin-, Pyridazin-, Piperazin-, Triazol-, und Triazinpuffer, bzw. Derivate oder Mischungen davon. Besonders bevorzugt ist dabei ein Imidazolpuffersystem. Als Lösungsmittel kommen je nach Puffersystem bevorzugt Mischungen von Alkoholen, typischerweise verzweigten oder unverzweigten C1 bis C6-Alkoholen, bzw. von Gylcerin mit Wasser in Frage.

Besonders bevorzugt ist ein wäßrig ethanolischer Imidazolpuffer. Die Konzentration an Imidazol in der Tränklösung sollte im Bereich von 0,1 - 1,0 mmol/l, bevorzugterweise im Bereich 0,3 - 0,4 mmol/l liegen. Das Verhältnis von Wasser zu Ethanol sollte im Mischungsverhältnis 1:4 bis 4:1 liegen. Besonders bevorzugt ist ein Verhältnis von 1:3.

Die Konzentration an o-CPC in der Pufferlösung sollte im Bereich von 1 - 20 mmol/l, bevorzugterweise im Bereich 2 - 5 mmol/l liegen.

Durch gezielte Einstellung des pH-Wertes obiger Puffersysteme lassen sich Bedingungen einstellen, bei denen o-CPC gegenüber Ca und Mg eine identische molare Empfindlichkeit besitzt. Im Falle des bevorzugten wäßrig ethanolischen Imidazolpuffers sollte ein pH-Wert von 6,75 eingestellt werden (siehe Beispiel 3). Ein Fachmann ist in der Lage, durch Bestimmung der jeweiligen Extinktionswerte geeignete pH-Werte für andere Puffersysteme zu finden.

Als besonders vorteilhaft hat sich bei Verwendung der erfindungsgemäß bevorzugten Puffersysteme erwiesen, daß o-CPC nicht, wie nach dem Stand der Technik beschrieben, zunächst in stark saurem Medium gelöst werden muß, sondern direkt in der Pufferlösung aufgelöst werden kann.

Von wesentlichem Vorteil für ein Testsystem ist seine lange Lagerfähigkeit ohne Änderung der Testeigenschaften (insbesondere Kalibrationsdaten). Häufig werden deshalb Stabilisatoren in ein Testsystem integriert. Daher kann gegebenenfalls auch die Tränklösung zur Herstellung des erfindungsgemäßen Teststreifens einen Stabilisator enthalten. Stabilisatoren, wie z.B. Alkylpolyalkylenglycolether, Hydroxypropylcellulose oder Polyalkohole, sind dem Fachmann bekannt. Besonders bevorzugt wird dem erfindungsgemäßen Teststreifen ein Bariumsalz als Stabilisator zugesetzt, da gefunden wurde, daß durch Zusatz von Spuren eines Bariumsalzes (besonders bevorzugt Bariumchlorid) zu der Tränklösung nicht nur eine deutliche Stabilisierung des Testsystems (Haltbarkeit bei Raumtemperatur > 2 Jahre), sondern gleichzeitig eine Verbesserung der Homogenität der Färbung der Testzonen erreicht werden kann. Dies hat eine höhere Bestimmungsgenauigkeit und Reproduzierbarkeit der Bestimmung zur Folge. Die Konzentration an Bariumsalzen, bevorzugt Bariumchlorid, in der Tränklösung sollte im Bereich von 0,05 - 1,0 mmol/l, bevorzugterweise im Bereich 0,1 - 0,2 mmol/l liegen (siehe Beispiel 1).

Durch Einbringen eines geeigneten Komplexbildners in den erfindungsgemäßen Teststreifen und Einstellung eines für das jeweilige Element optimalen pH-Wertes kann das erfindungsgemäße Nachweissystem auch zur getrennten Bestimmung von Ca bzw. Mg eingesetzt werden. Die Bestimmung eines geeigneten pH-Werts sowie geeignete Komplexbildner sind dem Fachmann bekannt. Als besonders vorteilhaft hat sich zur Kompexierung von Mg 8-Hydroxychinolin bzw. 8-Hydroxychinolinsulfonsäure, für Ca [Bis-(aminoethyl)-glycolether-N,N,N',N'-tetraessigsäure (=Titriplex VI) erwiesen (siehe Beispiele 4 und 5).

Durch Einbringen eines in Bezug auf Ca und Mg unspezifischen Komplexbildners in das Nachweissystem, wie z.B. Titriplex III (Ethylen-dinitrilotetraessigsäure Dinatriumsalz) kann außerdem auch auf die Sensitivität der Bestimmung Einfluß genommen werden, d.h. die Bestimmung weniger sensitiv gestaltet werden. Durch Kombination zweier Testzonen unterschiedlicher Empfindlichkeit kann dadurch der Meßbereich des Teststreifens entsprechend vergrößert werden. Dies ist besonders für die Bestimmung von Gesamthärte von großer Bedeutung, da Wässer sehr unterschiedliche Härtegrade aufweisen können (Bereich ca. 0,5 - 30 °dH) und ein Testsystem diesen Meßbereich erfassen sollte. (siehe Beispiel 2) Als saugfähige Träger für die erfindungsgemäßen Teststreifen können alle Materialien verwendet werden, die üblicherweise für solche trockenchemischen Tests im Gebrauch sind und die frei von Calcium und Magnesium sind. Am weitesten verbreitet ist die Verwendung von Filterpapier, jedoch können auch andere saugfähige Cellulose- oder Kunststoffprodukte eingesetzt werden.

Die saugfähigen Träger werden in bekannter Weise mit Tränklösungen, bevorzugt dem Puffersystem, imprägniert, die alle zur Bestimmung von Gesamthärte, Calcium bzw. Magnesium notwendigen Reagenzien enthalten. Die getränkten und getrockneten Papiere können geeignet zugeschnitten und in bekannter Weise zur Herstellung der Teststreifen auf Trägerfolien aufgeklebt bzw. aufgesiegelt werden.

Zur Durchführung des erfindungsgemäßen Verfahrens wird ein Teststreifen, der zumindest eine Zone mit o-CPC in einem Puffer mit pH < 8,5 enthält, kurz in die zu analysierende Probe getaucht bzw. mit der zu analysierenden Probe benetzt. Anschließend wird gegebenenfalls überschüssige Probenlösung abgestreift. Innerhalb kurzer Zeit (zumeist < 1 Minute) tritt eine Farbentwicklung auf, die über den Analysezeitraum stabil bleibt und visuell oder reflektometrisch analysiert werden kann.

Das erfindungsgemäße Verfahren bzw. die erfindungsgemäßen Teststreifen eignen sich insbesondere zum Einsatz in Analysekits. Ein erfindungsgemäßer Analysekit enthält zumindest einen erfindungsgemäßen Teststreifen zur Bestimmung des Gesamtgehaltes an Ca und Mg in einer wässrigen Probe. Optional kann der Analysekit weitere Bestandteile wie z.B. eine Beschreibung des Verfahrensablaufs oder eine Farbtafel zur visuellen Auswertung enthalten.

Somit bietet das erfindungsgemäße Verfahren eine einfache, schnelle und empfindliche Methode zum Nachweis von Mg und Ca in wässrigen Proben. Durch die einfache Verfahrensdurchführung kann der Nachweis ohne apparativen Aufwand und ohne Handhabung von giftigen Chemikalien auch von ungeübtem Personal durchgeführt werden. Da auch für einen quantitativen Nachweis nur eine, zur Abdeckung eines großen Konzentrationsbereiches meist zwei, Nachweiszonen auf dem Teststreifen notwendig sind, ist das erfindungsgemäße Nachweisprinzip problemlos auch in Mehrfachteststreifen integrierbar. Es läßt sich also z.B. kombinieren mit Nachweiszonen für Nitrat, Nitrit, pH und/oder Carbonathärte.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Beispiel 1

### Bestimmung von Gesamthärte mit Testsstreifen - reflektometrische Auswertung der Reaktionsfarbe:

### Herstellung der Teststäbchen:

Auf ein Filterpapier (Fa. Binzer, 1450 CV; säuregewaschen) wird nachfolgende Tränklösung aufgebracht und danach mit warmer Luft getrocknet. Das Papier wird mit Schmelzkleber (z.B. Kleber Dynapol 1272) auf eine weiße Trägerfolie aufgesiegelt und geeignet in Streifen geschnitten, so daß eine Reaktionszone von ca. 6 mm x 8 mm resultiert.

### Zusammensetzung der Tränklösung:

In 100 ml Ethanol, absolut, werden nacheinander 0,3 g o-CPC, 3,3 g Imidazol eingewogen und unter Rühren gelöst. Anschließend werden 30 ml Wasser und 1 ml 0,1%iger Bariumchloridlösung zugegeben. Der pH der Lösung wird mit Salzsäure, 1 mol/l, auf pH 6,75 eingestellt.

### Herstellung der Standardlösungen:

Wäßrige Standardlösungen werden durch Einwiegen eines geeigneten Calcium- bzw. Magnesium-Salzes, vorzugsweise Calciumchlorid-dihydrat bzw. Magnesiumnitrat-hexahydrat hergestellt.

### Analyse:

Die Teststäbchen werden für die Bestimmung kurzzeitig in die Probelösung eingetaucht, so daß sie vollständig benetzt werden. In Abhängigkeit vom Grad an Gesamthärte entstehen innerhalb weniger Sekunden unterschiedlich intensive, über einen längeren Zeitraum stabile Violettfärbungen, die bereits nach 15 sek visuell durch Vergleich mit einer Farbkarte ausgewertet werden können.

Zur quantitativen Auswertung werden die Teststreifen nach exakt 15 Sekunden in einem kleinen Handreflektometer auf Diodenbasis (Reflektometer RQflex^{®}) ausgewertet. Den Zusammenhang zwischen der gemessenen relativen Remission (%) und dem Gesamthärtegrad zeigt Tabelle 1.

**Tabelle 1**

| **°dH** | **% Rem** |
|---|---|
| 0 | 70 |
| 1,4 | 62 |
| 1,8 | 58 |
| 4,2 | 53 |
| 5,6 | 49 |
| 7,0 | 45 |
| 8,4 | 41 |
| 9,8 | 37 |
| 12,6 | 30 |

### Beispiel 2

### Kombination zweier Reaktionszonen mit unterschiedlichen Nachweisempfindlichkeiten zur Erhöhung des Meßbereiches am Beispiel von Gesamthärte.

Auf die Teststäbchen aus Beispiel 1 wurde eine zusätzliche Zone aufgebracht, die mit nachfolgender Reagenzlösung getränkt wurde. Der Abstand der beiden 8 x 6 mm großen Testzonen betrug 4 mm.

### Zusammensetzung der Tränklösung für Zone 2:

Zu 70 ml der Tränklösung für Zone 1 (siehe Beispiel 1) werden 2,2 ml einer 0,1 molaren Titriplex III -Lösung (Ethylen-dinitrilotetraessigsäure Dinatriumsalz) gegeben. Der pH der Lösung wird mit Salzsäure, 1 mol/l, auf pH 6,75 eingestellt.

### Analyse:

Die Teststreifen wurden wie unter Beispiel 1 nach exakt 15 Sekunden im Reflektometer RQflex^{®} vermessen. Das Reflektometer RQflex^{®} besitzt eine, für diese Art der Auswertung notwendige Doppeloptik.
Den Zusammenhang zwischen der gemessenen relativen Remission (%) und dem Gesamthärtegrad für beide Zonen zeigt Tabelle 2.

**Tabelle 2:**

| **°dH** | **Zone 1 [% Rem]** | **°dH** | **Zone 2 [% Rem]** |
|---|---|---|---|
| 0 | 70 | 11,2 | 64 |
| 1,4 | 62 | 12,6 | 59 |
| 1,8 | 58 | 14,0 | 53 |
| 4,2 | 53 | 15,4 | 48 |
| 5,6 | 49 | 17,5 | 42 |
| 7,0 | 45 | 21,0 | 36 |
| 8,4 | 41 | 25,2 | 30 |
| 9,8 | 37 | 28,0 | 27 |
| 12,6 | 30 | 30,0 | 26 |

Durch Kombination der beiden Testzonen läßt sich Gesamthärte quantitativ im Bereich von ca. 0,1 - 30 °dH bestimmen.

### Beispiel 3

### Einfluß des pH-Wertes der Tränklösung auf die Nachweisempfindlichkeit des Systems gegenüber Ca und Mg.

Die Tränklösung aus Beispiel 1 wurde mit HCl auf verschiedene pH-Werte (pH 6,75 und 7,1) eingestellt und entsprechend Teststäbchen hergestellt. Anschließend wurden Ca-Standards und Mg-Standards gleicher Molarität vermessen.

Den Zusammenhang zwischen der gemessenen relativen Remission (%) und der Ca- bzw. Mg-Konzentration in Abhängigkeit vom pH der Tränklösung zeigen die Tabellen 3 und 4.
Nur bei einem pH-Wert der Tränklösung von 6,75 konnte für Ca und Mg eine gleiche molare Empfindlichkeit erhalten werden, was Voraussetzung für die Bestimmung der Summe an Ca und Mg ist.

**Tabelle 3:**

| **Tränklösung mit pH 6,75** | | | | |
|---|---|---|---|---|
| **Mmol/l** | **Entspricht** | **Entspricht** | **Ca** | **Mg** |
| **Ca bzw. Mg** | **mg/l Ca** | **mg/l Mg** | **[% Rem]** | **[% Rem]** |
| **0** | 0 | 0 | 69,5 | 69,5 |
| **0,25** | 10 | 6 | 63,0 | 62,2 |
| **0,5** | 20 | 12 | 55,8 | 55,3 |
| **1,25** | 50 | 30 | 44,1 | 43,4 |
| **2,5** | 100 | 60 | 29,5 | 28,4 |
| **3,75** | 150 | 90 | 23,9 | 22,8 |
| **5,0** | 200 | 120 | 20,4 | 20,0 |

**Tabelle 4:**

| **Tränklösung mit pH 7,1** | | | | |
|---|---|---|---|---|
| **mmol/l Ca** | **Entspricht** | **Entspricht** | **Ca** | **Mg** |
| **bzw. Mg** | **mg/l Ca** | **mg/l Mg** | **[% Rem]** | **[% Rem]** |
| **0** | | | 69,0 | 68,5 |
| **0,5** | 20 | 12 | 48,5 | 53,9 |
| **1,25** | 50 | 30 | 26,3 | 32,6 |
| **2,5** | 100 | 60 | 16,2 | 21,3 |
| **3,75** | 150 | 90 | 13,8 | 18,4 |

### Beispiel 4

### Bestimmung von Magnesium mit Testsstreifen - reflektometrische Auswertung der Reaktionsfarbe:

Zu der Tränklösung aus Beispiel 1 wurde zur Komplexierung von Calcium Titriplex VI zugegeben. Die Konzentration an Titriplex VI in der Tränklösung betrug 7,5 g/l. Der pH-Wert der Lösung wurde mit HCl auf 7,0 eingestellt. Die Zugabe an Titriplex VI ermöglicht eine störungsfreie Analyse von Mg in Gegenwart von bis zu 200 mg/l Ca.

Den Zusammenhang zwischen der gemessenen relativen Remission (%) und dem Magnesiumgehalt zeigt Tabelle 5

**Tabelle 5:**

| **mg/l Mg** | **% Rem** |
|---|---|
| 0 | 68 |
| 5 | 60 |
| 10 | 54 |
| 25 | 43 |
| 50 | 33 |
| 75 | 26 |

### Beispiel 5

### Bestimmung von Calcium mit Testsstreifen - reflektometrische Auswertung der Reaktionsfarbe:

Zu der Tränklösung aus Beispiel 1 wurde zur Komplexierung von Magnesium 8-Hydroxychinolin-5-sulfonsäure zugegeben. Die Konzentration an 8-Hydroxychinolin-5-sulfonsäure in der Tränklösung betrug 11,8 g/l. Der pH-Wert der Lösung wurde mit HCl auf 7,4 eingestellt. Die Zugabe an Titriplex VI ermöglicht eine störungsfreie Analyse von Ca in Gegenwart von bis zu 100 mg/l Mg.
Den Zusammenhang zwischen der gemessenen relativen Remission (%) und dem Calciumgehalt zeigt Tabelle 6.

**Tabelle 6**

| **mg/l Ca** | **% Rem** |
|---|---|
| 0 | 70 |
| 10 | 65 |
| 25 | 54 |
| 50 | 47 |
| 75 | 40 |
| 100 | 31 |

### Beispiel 6

### Praxistest: Bestimmung von Gesamthärte, Calcium und Magnesium in Wasserproben-Vergleich mit Standardverfahren.

Es wurden 30 Trinkwasserproben aus dem öffentlichen Versorgungsnetz verschiedener Gemeinden in Niedersachsen zur Untersuchung herangezogen.

Als Standardverfahren zur Bestimmung von Mg wurde die Atomabsorptionsspektroskopie (s. Tab. 7), zur Bestimmung von Ca die Flammenphotometrie (s. Tab. 8) eingesetzt. Aus den Einzelwerten für Calcium und Magnesium wurde die Gesamthärte (s. Tab. 9) berechnet. Die Bestimmung der Gesamthärte erfolgte massanalytisch. Gemäß DIN 38409 Teil 6 wurde die Gesamthärte komplexometrisch mit Titriplex^{®} III-Lösung (Ethylen-dinitrilotetraessigsäure Dinatriumsalz) gegen eine Indikatorpuffertablette bestimmt (s. Tab. 9).

Für die reflektometrischen Analysen wurden die in den vorhergehenden Beispielen beschriebenen Teststäbchen eingesetzt. Zur Bestimmung von Gesamthärte die Teststäbchen aus Beispiel 2 (s. Tab. 9), zur Bestimmung von Magnesium die Teststäbchen aus Beispiel 4 (s. Tab. 7) und zur Bestimmung von Calcium die aus Beispiel 5 (s. Tab. 8).

**Tabelle 7:**

| Bestimmung von Mg-Ionen in Trinkwasserproben (mg / l) Vergleich zwischen AAS und Teststäbchen mit refl. Auswertung | | |
|---|---|---|
| **Probe** | **AAS** | **Teststäbchen** |
| 1 | 10 | 7 |
| 2 | 10 | 6 |
| 3 | 5 | <5 |
| 4 | 38 | 40 |
| 5 | 10 | 7 |
| 6 | 9 | 5 |
| 7 | 4 | <5 |
| 8 | 35 | 35 |
| 9 | 5 | 3 |
| 10 | 33 | 30 |
| 11 | 5 | < 5 |
| 12 | 38 | 38 |
| 13 | 30 | 29 |
| 14 | 35 | 35 |
| 15 | 46 | 44 |
| 16 | 34 | 38 |
| 17 | 35 | 33 |
| 18 | 19 | 16 |
| 19 | 10 | 9 |
| 20 | 7 | 7 |
| 21 | 7 | < 5 |
| 22 | 5 | < 5 |
| 23 | 28 | 28 |
| 24 | 17 | 16 |
| 25 | 5 | < 5 |
| 26 | 40 | 39 |
| 27 | 5 | < 5 |
| 28 | 6 | < 5 |
| 29 | 5 | < 5 |
| 30 | 9 | < 5 |

**Tabelle 8:**

| Bestimmung von Ca-Ionen in Trinkwasserproben (mg / l) Vergleich zwischen Flammenphotometrie und Teststäbchen mit reflektometrischer Auswertung | | |
|---|---|---|
| **Probe** | **Flammenphotomtrie** | **Teststäbchen** |
| 1 | 77 | 71 |
| 2 | 158 | 124 |
| 3 | 50 | 41 |
| 4 | 112 | 105 |
| 5 | 95 | 89 |
| 6 | 62 | 54 |
| 7 | 62 | 54 |
| 8 | 83 | 87 |
| 9 | 72 | 73 |
| 10 | 95 | 101 |
| 11 | 99 | 100 |
| 12 | 84 | 88 |
| 13 | 73 | 79 |
| 14 | 77 | 78 |
| 15 | 128 | 109 |
| 16 | 57 | 54 |
| 17 | 77 | 79 |
| 18 | 150 | 124 |
| 19 | 48 | 49 |
| 20 | 23 | 18 |
| 21 | 43 | 39 |
| 22 | 40 | 48 |
| 23 | 97 | 102 |
| 24 | 77 | 79 |
| 25 | 39 | 34 |
| 26 | 114 | 112 |
| 27 | 45 | 40 |
| 28 | 44 | 40 |
| 29 | 41 | 40 |
| 30 | 43 | 44 |

**Tabelle 9:**

| Massanalytische und reflektometrische (mit Teststäbchen) Bestimmung der Gesamthärte (°dH) in Trinkwasserproben, sowie berechnete Werte aus den Ca- und Mg-Bestimmungen | | | |
|---|---|---|---|
| **Probe** | **Massanalyse** | **Teststäbchen** | **Berechnet** |
| 1 | 12,4 | 13,0 | 13,1 |
| 2 | 22,7 | 23,0 | 24,4 |
| 3 | 7,5 | 9,0 | 8,2 |
| 4 | 22,9 | 22,8 | 24,5 |
| 5 | 14,7 | 14,4 | 15,6 |
| 6 | 10,1 | 10,6 | 10,8 |
| 7 | 9,2 | 10,6 | 9,6 |
| 8 | 18,6 | 18,8 | 19,7 |
| 9 | 11,1 | 12,1 | 11,3 |
| 10 | 20,1 | 19,8 | 20,9 |
| 11 | 14,7 | 14,5 | 15,1 |
| 12 | 20,2 | 19,5 | 20,6 |
| 13 | 16,6 | 16,7 | 17,1 |
| 14 | 18,4 | 17,7 | 18,9 |
| 15 | 26,5 | 25,3 | 28,5 |
| 16 | 15,5 | 14,9 | 15,8 |
| 17 | 18,4 | 19,3 | 18,9 |
| 18 | 23,7 | 23,7 | 25,4 |
| 19 | 8,9 | 9,7 | 9,0 |
| 20 | 4,7 | 5,3 | 4,8 |
| 21 | 7,1 | 7,1 | 7,6 |
| 22 | 6,3 | 6,9 | 6,8 |
| 23 | 18,9 | 18,7 | 20,1 |
| 24 | 13,9 | 13,9 | 14,7 |
| 25 | 6,2 | 6,3 | 6,7 |
| 26 | 23,8 | 23,9 | 25,2 |
| 27 | 7,0 | 7,5 | 7,5 |
| 28 | 6,9 | 6,3 | 7,6 |
| 29 | 6,5 | 7,1 | 7,1 |
| 30 | 6,9 | 8,0 | 7,6 |

## Patentansprüche

1. Verfahren zur Bestimmung der Summe aus Calcium und Magnesium in wässrigen Proben, wonach ein Teststreifen, auf den Orthokresolphtalein-Komplexon als farbgebendes Reagenz in einem Puffersystem mit einem pH-Wert unter 8,5 aufgebracht ist, mit der zu analysierenden Probe benetzt wird und die resultierende Farbreaktion reflektometrisch oder visuell ausgewertet wird, wobei der pH-Wert des Puffersystems so eingestellt ist, dass Orthokresolphtalein-Komplexon gegenüber Calcium und Magnesium eine identische molare Empfindlichkeit besitzt.

2. Verfahren nach Anspruch 1, **dadurch gekennzeichnet, dass** der pH-Wert des Puffersystems des eingesetzten Teststreifens kleiner oder gleich pH 7 ist.

3. Teststreifen zum Nachweis der Summe aus Calcium und Magnesium in wässrigen Proben, der mindestens eine Zone aufweist, die zumindest Orthokresolphtalein-Komplexon in einem Puffersystem mit pH < 8,5 enthält, wobei der pH-Wert des Puffersystems so eingestellt ist, dass Orthokresolphtalein-Komplexon gegenüber Calcium und Magnesium eine identische molare Empfindlichkeit besitzt.

4. Teststreifen nach Anspruch 3, **dadurch gekennzeichnet, dass** das Puffersystem aus einem Imidazolpuffer, pH 6,75, besteht.

5. Teststreifen nach einem der Ansprüche 3 oder 4, **dadurch gekennzeichnet, dass** zumindest eine Zone des Teststreifens zusätzlich ein Bariumsalz enthält.

6. Teststreifen nach einem der Ansprüche 3 bis 5, **dadurch gekennzeichnet, dass** zumindest eine Zone des Teststreifens einen Ca- oder Mg- spezifischen oder einen unspezifischen Komplexbildner enthält.

7. Teststreifen nach einem der Ansprüche 3 bis 6, **dadurch gekennzeichnet, dass** der Teststreifen zusätzlich mindestens eine weitere Zone zum Nachweis anderer Analyten aufweist.

8. Analysekit zum Nachweis von Ca und Mg in wässrigen Proben, der zumindest einen Teststreifen entsprechend einem der Ansprüche 3 bis 7 enthält.

## Claims

1. Method for the determination of the sum of calcium and magnesium in aqueous samples, in which a test strip to which orthocresolphthalein complexon as colouring reagent in a buffer system having a pH of below 8.5 has been applied is wetted with the sample to be analysed, and the resultant colour reaction is evaluated reflectometrically or visually, where the pH of the buffer system has been set so that the orthocresolphthalein complexon has identical molar sensitivity to calcium and magnesium.

2. Method according to Claim 1, **characterised in that** the pH of the buffer system of the test strip employed is less than or equal to pH 7.

3. Test strip for the determination of the sum of calcium and magnesium in aqueous samples, which has at least one zone which contains at least orthocresolphthalein complexon in a buffer system of pH < 8.5, where the pH of the buffer system has been set so that the orthocresolphthalein complexon has identical molar sensitivity to calcium and magnesium.

4. Test strip according to Claim 3, **characterised in that** the buffer system consists of an imidazole buffer, pH 6.75.

5. Test strip according to one of Claims 3 or 4, **characterised in that** at least one zone of the test strip additionally contains a barium salt.

6. Test strip according to one of Claims 3 to 5, **characterised in that** at least one zone of the test strip contains a Ca- or Mg-specific or non-specific complexing agent.

7. Test strip according to one of Claims 3 to 6, **characterised in that** the test strip additionally has at least one further zone for the determination of other analytes.

8. Analytical kit for the determination of Ca and Mg in aqueous samples, which contains at least one test strip corresponding to one of Claims 3 to 7.

## Revendications

1. Méthode de détermination de la somme de calcium et de magnésium dans des échantillons aqueux, dans laquelle une bandelette réactive à laquelle on a appliqué un complexon d'orthocrésolphtaléine comme réactif colorant dans un système tampon ayant un pH inférieur à 8,5 est mouillée par l'échantillon à analyser, et la réaction colorée résultante est évaluée de manière réflectométrique ou visuelle, où le pH du système tampon a été fixé de sorte que le complexon d'orthocrésolphtaléine possède une sensibilité molaire identique au calcium et au magnésium.

2. Méthode selon la revendication 1, **caractérisée en ce que** le pH du système tampon de la bandelette réactive employée est inférieur ou égal à pH 7.

3. Bandelette réactive pour la détermination de la somme de calcium et de magnésium dans des échantillons aqueux, ayant au moins une zone contenant au moins du complexon d'orthocrésolphtaléine dans un système tampon de pH < 8.5, où le pH du système tampon a été fixé de sorte que la complexon d'orthocrésolphtaléine possède une sensibilité molaire identique au calcium et au magnésium.

4. Bandelette réactive selon la revendication 3, **caractérisée en ce que** le système tampon est constitué d'un tampon imidazole, pH 6,75.

5. Bandelette réactive selon l'une des revendications 3 ou 4, **caractérisée en ce qu'**au moins une zone de la bandelette réactive contient en outre un sel de baryum.

6. Bandelette réactive selon l'une des revendications 3 à 5, **caractérisée en ce qu'**au moins une zone de la bandelette réactive contient un agent complexant spécifique ou non spécifique de Ca ou Mg.

7. Bandelette réactive selon l'une des revendications 3 à 6, **caractérisée en ce que** la bandelette réactive contient en outre une zone supplémentaire pour la détermination d'autres analytes.

8. Kit analytique de détermination de Ca et de Mg dans des échantillons aqueux, contenant au moins une bandelette réactive correspondant à l'une des revendications 3 à 7.
